# EUROPÄISCHE PATENTSCHRIFT

(11) **EP 0 753 306 B1**
(45) Veröffentlichungstag und Bekanntmachung des Hinweises auf die Patenterteilung: **05.08.1998**
(21) Anmeldenummer: 96110306.6
(22) Anmeldetag: 26.06.1996
(51) Int. Cl.: A61K 35/78, F26B 7/00, C11B 9/02

(54) **Verfahren zur Herstellung von pharmazeutischen Zubereitungen mit höherem Gehalt an etherischen Ölen und Phenolen**
Process of obtention of pharmaceutical preparations with high content in essential oils and phenols
Procédé d'obtention de préparations pharmaceutiques à haute teneur en huiles essentielles et phénols

(30) Priorität: 10.07.1995 DE 19525026
(43) Veröffentlichungstag der Anmeldung: 15.01.1997
(73) Patentinhaber: Plantamed Arzneimittel GmbH, 92318 Neumarkt (DE)
(72) Erfinder: Joseph, Heinz W., Dr., 71522 Backnang (DE)
(74) Vertreter: Sandmair, Kurt, Dr. Dr.

(56) Entgegenhaltungen:
- EP-A- 0 435 302
- WO-A-95/03380
- FR-A- 2 699 088

## Beschreibung

Die Erfindung betrifft Verfahren zur Herstellung von Pflanzentrockenextrakten und diese enthaltende pharmazeutische Zubereitungen mit hohem Anteil an etherischen Ölen und Phenolen. Es ist bekannt, daß etherische Öle und Phenole, wie sie in Pflanzen vorkommen, entzündungshemmende, bakteriostatische, hyperämisierende und sekretolytische Wirkung haben (siehe z.B. ***Pharmazie 29 (5) 339, 1974; Journal of Ethnopharmacology 39, 167-170, 1993; Plantes Medicinales et Phytotherapie, 22 (3), 195-202, 1988).*** Diese Wirkungen sind in vielen Fällen linear dosisabhängig.

Das DAB 10 (Deutsches Arzneibuch, 10. Ausgabe) setzt Mindesgehalte dieser etherischen Öle und Phenole für die Drogenqualität fest, um dadurch eine Sicherheit bei der Therapie zu erhalten.

Am Beispiel Herba Thymi (Thymian), Stammpflanze Thymus vulgaris L., sind die Anforderungen im DAB 10 festgeschrieben. Nach DAB 10 muß Herba Thymi (eingesetzte Droge) mindestens 1,2 % etherisches Öl und mindestens 0,5 % Phenol enthalten, das als Thymol berechnet wird.

Die Gleichförmigkeit der Extrakte und der hohe Gehalt an etherischen Ölen ist ein wichtiges Kriterium für die Fertigware. Etherische Öle sind aufgrund der physikalischen Eigenschaften Substanzen mit extrem hoher Flüchtigkeit und Oxidierbarkeit. Phenole unterliegen ebenfalls einer leichten Oxidierbarkeit.

Aus diesem Grunde besteht eine hohe Gefahr, daß die Substanzen bei der Herstellung von Zubereitungen leicht zersetzt werden und durch die Oxidierbarkeit der Verbindungen, die viele Doppelbindungen enthalten können, die Wirksamkeit der pharmazeutischen Zubereitung leidet.

Die bislang in der pharmazeutischen Industrie benutzten, gängigen Trocknungsverfahren umfassen neben der klassischen Abdestillation und Trocknung unter Normalbedingungen auch Verfahren, die sich der Variation von Druck und Temperatur bedienen, um zu Trockenextrakten zu gelangen.

Das klassische Verfahren zur Herstellung von Trockenextrakten ist die Herstellung über einen Fluidextrakt oder eine Tinktur; nach anschließender Abdestillation der Lösungsmittel wird dann ein Spissumextrakt (zähflüssiger Extrakt) erreicht, dem häufig Hilfsstoffe und/oder Zusatzstoffe wie z.B. Lactose, Polyvinylpyrrolidon, Saccharose, Siliciumdioxid usw. zugesetzt werden. Diese feuchte, zähe Masse wird dann in Hordenschränken oder Trocknern zur gewünschten Trockenextraktzubereitung gebracht. Durch die Abdestillation unter Normalbedingungen, die hohen Temperaturen zur Endtrocknung und den mangelnden Sauerstoffabschluß werden hier bekannterweise sehr viele Nebenprodukte gefunden, die aufgrund dieses Herstellungsprozesses entstehen und möglicherweise eine nachteilige Wirkung besitzen.

Ein Verfahren, das sehr häufig in der Trockenextraktherstellung benutzt wird, ist das sog. Vakuumbandtrocknungsverfahren. Hierbei wird der Spissumextrakt nach einer Vortrocknung über Fallstromverdampfer zur Trockenextraktzubereitung gebracht. Die hier vorliegenden Temperaturen, Druckverhältnisse und Materialdurchlaufparameter liefern Extrakte mit geringerem Gehalt an etherischen Ölen und Phenolen. Bei diesem Verfahren liegen die Temperaturen zwischen 50°C und 105°C, der Druck bei ca. 18 mbar. Auch diese Verfahren verursachen Verluste an phenolischen Substanzen und etherischen Öl-Substanzen. Dies liegt unter anderem in der fehlenden Bewegung des zu trocknenden Produktes begründet (z.B. Diffusionseffekte).

Das Trocknungsverfahren mittels eines Wirbelschichttrockners benötigt Temperaturen zwischen ca. 47°C und 117°C. Trotz Einblasens von Stickstoff (Reduktion des Sauerstoffgehaltes) wird immerhin noch ein Rest von ca. 5 % Sauerstoff gefunden, wodurch verschiedene nachteilige Prozesse in Gang gesetzt werden. Die Trocknung in diesem Verfahren wird unter normalen Druckbedingungen durchgeführt. Dies erklärt die geringeren Gehalte an phenolischen Substanzen und etherischen Ölen. Dabei kann ebenfalls der Parameter Temperatur variiert werden, was nicht zur Erhöhung von Ausbeuten an etherischem Öl führt.

Aus der WO 95/3380 ist ein Kaltmazerationsverfahren zur Gewinnung von etherischen Ölen und Abtrennung von Terpenen bekannt, das aber nicht zur Herstellung von hochwertigen Pflanzentrockenextrakten mit hohem Anteil an phenolischen Inhaltsstoffen geeignet ist.

Aufgabe der vorliegenden Erfindung ist es deshalb, Verfahren zur Herstellung von Trockenextrakten, die aus etherisch ölhaltigen und phenolhaltigen Drogen hergestellt werden, zu schaffen, die die oben geschilderten Nachteile weitestgehend vermeiden.

Überraschend zeigt das erfindungsgemäße Herstellungsverfahren, bei dem man einen handelsüblichen, im folgenden, näher gekennzeichneten Vakuumtrockner der Fa. Inox Glatt, ehemals Inox Maurer einsetzt, daß in diesem System die Parameter Druck und Temperatur andere Auswirkungen haben als z.B. im Bereich der Vakuumbandtrocknung.

Das erfindungsgemäße Verfahren zur Herstellung von Pflanzenextrakten und sie enthaltende, pharmazeutische Zubereitungen mit hohem Anteil an etherischen Ölen und Phenolen umfaßt die Schritte des Einbringens eines über übliche und bekannte Weise erhaltenen Fluidextraktes oder einer Tinktur aus Drogen in das Trocknungssystem Inox® Maurer, bzw. Inox® Glatt. Von diesen handelsüblichen Geräten können beispielsweise die mit der Bezeichnung IUT 20, IUT 50, IUT 100 und IUT 2000 oder andere Typen aus der Baureihe IUT je nach benötigter Chargengröße verwendet werden.

Das Vakuumtrocknungssystem weist die folgenden Merkmale auf:
ein sich durch die zylindrische Misch- und Trocknerkammer erstreckendes mehrschenkliges Rührwerk mit eigenem Antrieb und je nach Erfordernissen Brüdenfilter, Rückspülungseinrichtung, Lösungsmittelkondensator mit Nachkühler und Auffanggefäß, Rückkondensator, eine Prozeß-, Steuer- und Regeleinheit, sowie ggf. Granulierdüsen. Sein besonderes Kennzeichen ist eine sich über die Gesamttiefe der Trockner- und Mischkammer erstreckender Zerhacker mit rührwerksunabhängigem Antrieb sowie ein kammartiger Stator zur Erhöhung der Zerhackerwirkung, durch den die Zerhackermesser hindurchrotieren.

Zu Beginn des Trocknungsprozesses wird eine Vor- und Rücklauftemperatur zwischen 120°C und 5°C eingestellt und bei einer Innenraumtemperatur zwischen 10°C und 80°C, bevorzugt zwischen 25°C und 70°C, sowie einer Brüdentemperatur zwischen 15°C und 55°C, getrocknet. Der Druck variiert zwischen 0,5 und 1000 mbar, bevorzugt zwischen 5 und 100 mbar, besonders bevorzugt zwischen 30 und 70 mbar. Die Trocknung muß bei einer Rührergeschwindigkeit zwischen 0 und 10 Upm, bevorzugt zwischen 2 und 5 Upm durchgeführt werden. Der Zerhacker hat eine Umdrehungsgeschwindigkeit bis zu 1200 Upm, bevorzugt zwischen 200 und 800 Upm. Die bevorzugte Brüdentemperatur beträgt 18°C bis 38°C bzw. 20°C bis 40°C. In Abhängigkeit des Produktes und der im Einzelfall verwendeten Parameter werden die Trocknungszeit und die Messungen zum Trocknungsverlust (TV) regelmäßig bestimmt (siehe Tabelle 1). Üblicherweise ist bei einem Trocknungsverlust zwischen 0 und 10 % und einer Trocknungszeit zwischen 4 und 24 Stunden der Trocknungsvorgang beendet.

Am Beispiel der Herstellung von Thymian-Trockenextrakt konnte nachgewiesen werden, daß im Vergleich zu den gängigen Verfahren des Standes der Technik mit dem erfindungsgemäßen Verfahren ein weitaus höherer Gehalt an etherischen Ölen und Phenolen gefunden wurde.

Dieser Befund war völlig überraschend, wie anhand des untenstehend beschriebenen Vergleichsversuches aufgezeigt wird.

Die Behandlung der flüssigen Extrakte nach dem erfindungsgemäßen Verfahren gewährleistet die bislang schonendste Herstellung von Trockenextrakten aus etherisch ölhaltigen Drogen. Alle übrigen Trocknungsverfahren ergeben weitaus höhere Verluste und somit eine Reduktion des möglichen therapeutischen Erfolges. Dies ist auch von wesentlichem Vorteil bei der wichtigen Standardisierung des Wirkstoffgehalts.

Außerdem bietet das erfindungsgemäße Verfahren, in Verbindung mit der besonderen Vorrichtung, die Möglichkeit, Lösungsmittel, die zur Herstellung von Fluidextrakten benutzt werden, schonend zurückzugewinnen und einer weiteren Verwertung zuzuführen. Selbst geringste Mengen werden nicht mehr an die Atmosphäre abgegeben, da durch die entsprechenden Kondensatoreinrichtungen am Trockner selbst die Lösungsmittel wieder zurückgewonnen werden können. Damit wird auch den strengen Umweltschutzauflagen Rechnung getragen, und zwar ohne daß komplizierte zusätzliche bzw. nachgeschaltete technische Maßnahmen oder Einrichtungen erforderlich wären.

Als weiterer Vorteil kommt hinzu, daß die physikalische Konsistenz der Produkte erhöht wird und selbst Trockenextrakte hergestellt werden können, die einen zähen, klebrigen, pastösen oder klumpenbildenden Zwischenzustand durchschreiten.

Ferner ist es als unerwarteter Vorteil anzusehen, daß der Trockenextrakt ohne Entnahme direkt im gleichen Trocknersystem durch nachfolgendes Vermischen mit einem oder mehreren weiteren Trockenextrakten oder anderen Extrakten und/oder Wirkstoffen, und/oder Granulieren, und/oder Verdünnen im gleichen Trocknungsgerät den soeben fertiggestellten Trockenextrakt zu den fertigen, pharmazeutischen Zubereitungen oder Halbfabrikaten kontinuierlich weiterverarbeitet.

### Beispiele:

Zur Herstellung der verschiedenen eingesetzten Tinkturen wurden verschiedene Chargen von Herba Thymi eingesetzt. Die aus der Droge erhaltene Tinktur wurde mit Ethanol 70 % (V/V) nach üblichen Verfahren hergestellt und der Gehalt an Phenolen bestimmt.

Der Gehalt an ehterischen Ölen wurde nur in der Ausgangsdroge ermittelt, da er analytisch aus der Tinkturform schwer bestimmbar ist.

Gegebenenfalls wurde nach Zusatz üblicher Zuschlagsstoffe (17 % Glukosesirup und 3 % Siliciumdioxid) ein Teil der Tinktur nach herkömmlichen Verfahren (Vakuumbandtrocknung und Wirbelschichttrocknung) und der andere Teil nach dem erfindungsgemäßen Verfahren getrocknet.

Für das erfindungsgemäße Verfahren wurden mehrere Versuche durchgeführt. Hierbei variierte die Menge eingesetzter Tinktur zwischen 50 und 2000 Litern. Die Tinktur wurde in Vakuumtrocknern der Firma Inox/Glatt bzw. Maurer (IUT 20, IUT 50, IUT 100, IUT 2000) bei Raumtemperatur eingesaugt und zwischen 120°C und 5°C Vorlauf- und Rücklauftemperatur getrocknet.

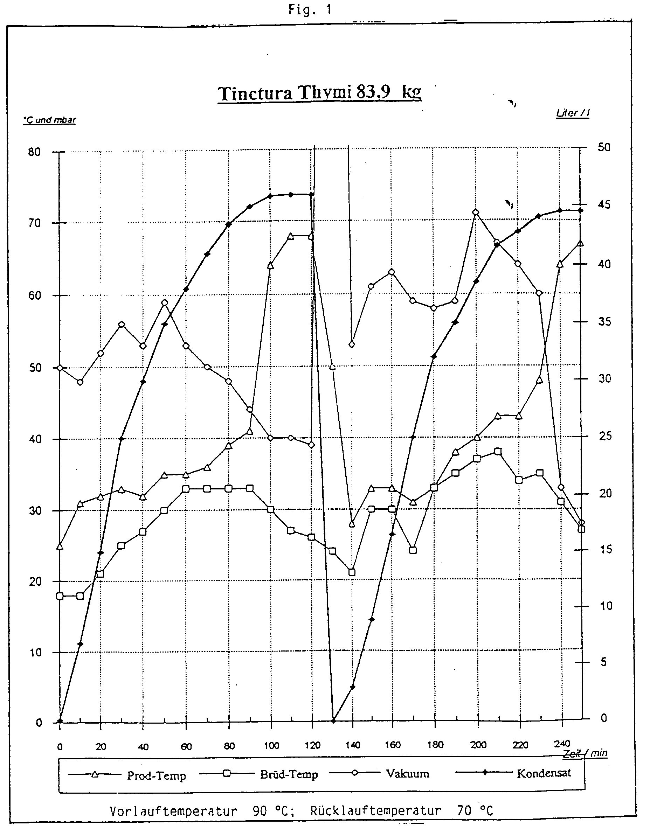

Die im Reaktionsraum vorliegende Temperatur lag während der Herstellung zwischen 25°C und 70°C, der Druck lag zwischen 30 und 70 mbar (Fig. 1).

Die Figur 1 zeigt die Verläufe der Produkttemperatur, der Filtertemperatur (Brüdentemperatur), der Druckverhältnisse und der Kondensatmenge, die bei der Trocknung des Thymian-Fluidextraktes gemäß des Ausführungsbeispieles an einer IUT-100-Anlage gemessen wurden.

Die Verdampfungsleistung lag zwischen 300 1/h und 330 1/h; sie unterliegt jedoch Schwankungen aufgrund der anfänglich leicht abdestillierenden Alkoholmenge im Vergleich zum schwerer flüchtigen Wasser. Nach Erreichen des gewünschten Trocknungs-verlustes (TV) wurde die getrocknete Substanz entnommen und der Trocknungsverlust (siehe Tab. 1), der Phenolgehalt (siehe Tab. 2) und die etherischen Öle (siehe Tab. 3) bestimmt. In den Tabellen 2 und 3 beziehen sich die %-Werte der Wiederfindungsrate bei etherischen Ölen, wie oben angemerkt, auf die Ausgangsdroge und die %-Werte für Phenole auf die Ausgangstinktur.

**Tabelle 1**

| Trocknungsverlust bzw. Trockenrückstand | |
|---|---|
| | TV bzw. TR |
| Thymian-Tinktur | 2,11 % |
| TE Inox-Maurer/Glatt nativ® (ohne Zuschlag) | 5,81 % |
| TE Inox-Maurer/Glatt® mit Zuschlagsstoffen (Glukosesirup, Siliciumdioxid) | 4,35 % |
| TE Wirbelschichttrocknung nativ (St.d.T./Vergleich) | 6,21 % |
| TE Vakuumbandtrocknung (St.d.T./Vergleich) | 2,92 % |
| TE = Trockenextrakt | |

**Tabelle 2**

| Phenolgehalt | | |
|---|---|---|
| | Phenolgehalt berechnet als Thymol | WDF |
| Thymian-Tinktur | 0,0935 % | --- |
| TE Inox-Maurer/Glatt® nativ (ohne Zuschlag) | 1,632 % | 37 % |
| TE Inox-Maurer/Glatt® mit Zuschlagsstoffen (Glukosesirup, Siliciumdioxid) | 1,262 % | 35 % |
| TE Wirbelschichttrockng. nativ (St.d.T./Vergleich) | 0,609 % | 12 % |
| TE Vakuumbandtrocknung (St.d.T./Vergleich) | 0,164 % | 4,5 % |
| TE = Trockenextrakt; WDF = Wiederfindungsrate | | |

**Tabelle 3**

| Etherisch-Öl-Gehalt | | |
|---|---|---|
| | Ätherisch-Öl-Gehalt | WDF |
| Thymian-Droge | 2,25 % | --- |
| TE Inox-Maurer/Glatt® nativ (ohne Zuschlag) | 3,08 % | 29 % |
| TE Inox-Maurer/Glatt® mit Zuschlagsstoffen (Glukosesirup, Siliciumdioxid) | 1,95 % | 22 % |
| TE Wirbelschichttrocknung nativ (St.d.T./Vergleich) | 1,25 % | 12 % |
| TE Vakuumbandtrocknung (St.d.T./Vergleich) | 0,35 % | 4,0 % |
| TE = Trockenextrakt; WDF = Wiederfindungsrate | | |

Wie die vorstehenden Tabellen 2 und 3 zeigen, wird durch das erfindungsgemäße Verfahren im Vergleich zu den bekannten Verfahren ein weitaus höherer Gehalt an etherischen Ölen und Phenolen erhalten.

Die Trocknung ist bei einem Einsatz von 84 kg Tinktur in ca. 4 Stunden beendet. Hierbei muß beachtet werden, daß Rührer und Zerhacker der Anlage den auftretenden zähen bzw. klumpigen Zwischenzustand durch Ihre Bewegung verhindern, so daß das befürchtete Verbacken des Trockners durch das Füllgut ausbleibt.

Das nach Beendigung des Trocknungsverfahrens erhaltene pulverförmige Produkt kann nach üblichen und in der pharmazeutischen Technik bzw. Galenik bekannten Verfahren zu den gewünschten Arznei- und Darreichungsformen weiterverarbeitet werden.

Das erfindungsgemäße Verfahren wurde bei der Trocknung weiterer Ausgangsmaterialien wie im vorigen Beispiel beschrieben, durchgeführt und damit vergleichbare Ergebnisse erzielt. Diese Drogen waren Echinacea (Purpursonnenhut), Agnus castus (Mönchspfeffer), Allium cepa (Zwiebel), Hippocastanus (Roßkastanie), Hedera helix (Efeu), Curcuma (Gelbwurz) und Galphimia glauca.

Außerdem wurden flüssige Mischextrakte, die aus verschiedenen getrockneten Drogen gemeinsam hergestellt wurden, mit dem erfindungsgemäße Verfahren getrocknet.

## Patentansprüche

1. Verfahren zur Herstellung von Pflanzentrockenextrakten mit hohem Anteil an etherischen Ölen und Phenolen, bei dem man einen Fluidextrakt oder eine Tinktur aus Drogen in ein Vakuumtrocknungssystem mit einem sich durch die zylindrische Misch- und Trocknerkammer erstreckenden mehrschenkligen Rührwerk mit eigenem Antrieb, sowie erforderlichenfalls jeweils versehen mit Brüdenfilter, Rückspülungseinrichtung, Lösungsmittelkondensator mit Nachkühler und Auffanggefäß, Rückkondensator und einer Prozeß-, Steuer- und Regeleinheit, sowie ggf. mit Granulierdüsen einbringt, dadurch gekennzeichnet, daß das zu trocknende Gut in dem mit einem sich über die Gesamttiefe der Trockner- und Mischkammer erstreckenden Zerhacker mit durch einen kammförmigen Stator hindurchrotierenden Messern ausgestatteten Trockner
bei einer Vor- und Rücklauftemperatur zwischen 120°C und 5°C, einer Innenraumtemperatur zwischen 10°C und 80°C, bevorzugt zwischen 25°C und 70°C, einer Brüdentemperatur von 15°C bis 55°C sowie einem Druck zwischen 0,5 und 1000 mbar, bevorzugt zwischen 5 und 100 mbar, besonders bevorzugt zwischen 30 und 70 mbar, getrocknet wird,
wobei die Trocknung bei laufendem Rührer zwischen 0 und 10 Upm, bevorzugt zwischen 2 und 5 Upm, und einer Zerhackerumdrehungsgeschwindigkeit zwischen 200 und 800 Upm vorgenommen wird.

2. Verfahren nach Anspruch 1, bei dem die Vor- und Rücklauftemperatur zwischen 90°C und 70°C, die Innenraumtemperatur zwischen 25°C und 70°C, die Brüdentemperatur (Abluftfiltertemperatur) 18°C und 38°C bzw. zwischen 20°C und 40°C und der Druck zwischen 30 und 70 mbar betragen, sowie der Rührer bei 5 Upm und der Zerhacker bei 400 Upm laufen.

3. Verfahren nach einem oder mehreren der vorstehenden Ansprüche, bei dem man durch nachfolgendes Vermischen mit einem oder mehreren weiteren Trockenextrakten und/oder anderen Extrakten und/oder Wirkstoffen, und/oder Granulieren, und/oder Verdünnen im gleichen Trocknungsgerät den soeben erhaltenen Trockenextrakt zu den fertigen pharmazeutischen Zubereitungen oder Halbfabrikaten kontinuierlich ohne Entnahme bzw. Zwischenlagerung weiterverarbeitet.

4. Verfahren nach den Ansprüchen 1 bis 3, bei dem die eingesetzte Droge Echinacea, Agnus castus, Allium cepa, Hedera helix, Hippocastanus, Curcuma, Galphimia glauca oder Herba Thymi ist.

5. Verfahren nach Anspruch 1 bis 4, bei dem ein Mischextrakt aus 2 oder mehreren Drogen hergestellt wird.

6. Pharmazeutische Zubereitungen mit hohem Anteil an etherischen Ölen und Phenolen, enthaltend einen Drogenextrakt, hergestellt nach einem Verfahren gemäß einem der Ansprüche 1 bis 5.

## Claims

1. Process for the preparation of dry plant extracts containing a high level of ether oils and phenols, in which a fluid extract or a tincture of drugs is introduced into a vacuum drying system having a separately driven, multiple-armed stirring device which extends through the cylindrical mixing and drying chamber, and being provided if necessary with a vapour filter, a back-flushing device, a solvent condenser having an after-cooler and a collection vessel, a re-condenser and a process, control and regulating unit, and optionally having granulating nozzles, characterised in that the product to be dried is dried in the dryer which is equipped with a chopper extending through the total depth of the drying and mixing chamber and having blades which rotate through a stator which takes the form of a comb, at a forward and reverse travel temperature of between 120°C and 5°C, an interior temperature of between 10°C and 80°C, preferably between 25°C and 70°C, a vapour temperature of from 15°C to 55°C and a pressure of between 0.5 and 1000 mbar, preferably between 5 and 100 mbar, particularly preferably between 30 and 70 mbar, wherein drying is carried out with the stirrer running at between 0 and 10 rpm, preferably between 2 and 5 rpm, and with the speed of revolution of the chopper being between 200 and 800 rpm.

2. Process according to Claim 1, in which the forward and reverse travel temperature is between 90°C and 70°C, the interior temperature is between 25°C and 70°C, the vapour temperature (exhaust air filter temperature) is 18°C and 38°C or is between 20°C and 40°C, and the pressure is between 30 and 70 mbar, and the stirrer runs at 5 rpm and the chopper at 400 rpm.

3. Process according to one or more of the preceding claims, in which in the same drying apparatus the newly obtained dry extract is processed further to the finished pharmaceutical preparations or to semi-finished products in continuous manner without removal or intermediate storage, by subsequent intermixing with one or more further dry extracts and/or other extracts and/or active ingredients, and/or granulation and/or dilution.

4. Process according to Claims 1 to 3, in which the drug used is Echinacea, Agnus castus, Allium cepa, Hedera helix, Hippocastanus, Curcuma, Galphimia glauca or herb thyme.

5. Process according to Claims 1 to 4, in which a mixed extract is prepared from 2 or more drugs.

6. Pharmaceutical preparations containing a high level of ether oils and phenols, which contain a drug extract and are prepared by a process according to one of Claims 1 to 5.

## Revendications

1. Procédé de fabrication d'extraits secs végétaux ayant une forte teneur en huiles essentielles et en phénols, dans lequel on charge un extrait liquide ou une teinture, constitué d'essences, dans un système de séchage sous vide avec un agitateur à plusieurs branches, ayant son propre système d'entraînement, s'étendant sur la profondeur totale de la chambre de séchage et de mélange cylindrique, et équipé si nécessaire, respectivement, d'un filtre pour la vapeur, d'un dispositif de lavage à contre-courant, d'un condenseur de solvants avec réfrigérant complémentaire et récipient de réception, d'un condenseur à reflux et d'une unité de traitement, de pilotage et de régulation, et éventuellement de buses de granulations, caractérisé en ce que, l'on sèche la substance à dessécher dans le système de séchage, équipé d'un hacheur à couteaux tournant à travers un stator en forme de peigne, s'étendant sur la profondeur totale de la chambre de séchage et de mélange à une température de tête et de queue de colonne entre 120°C et 5°C, une température à l'intérieur de la chambre entre 10°C et 80°C, de préférence entre 25°C et 70°C, une température de vapeur de 15°C jusqu'à 55°C, et une pression entre 0,5 et 1000 mbar, de préférence entre 5 et 100 mbar, de manière particulièrement préférée entre 30 et 70 mbar, ce par quoi on réalise le séchage avec l'agitateur tournant entre 0 et 10 tr/min., de préférence entre 2 et 5 tr/min., et avec une vitesse de rotation du hacheur entre 200 et 800 tr/min.

2. Procédé selon la revendication 1, dans lequel la température de tête et de queue de colonne se situe entre 90°C et 70°C, la température intérieure de la chambre entre 25°C et 70°C, la température de la vapeur (température du filtre d'évacuation d'air) entre 18°C et 38°C, ou entre 20°C et 40°C, et la pression entre 30 et 70 mbar, et dans lequel l'agitateur tourne à 5 tr/min. et le hacheur à 400 tr/min.

3. Procédé selon une ou plusieurs des revendications précédentes, dans lequel on transforme ensuite, par mélange en continu avec un ou plusieurs autres extraits secs et/ou autres extraits et/ou ingrédients, et/ou granulés, et/ou diluants, dans le même appareil de séchage l'extrait sec ainsi obtenu en préparations pharmaceutiques ou en produits semi-finis sans soutirage ni stockage intermédiaire.

4. Procédé selon les revendications 1 à 3, dans lequel on utilise les essences Echinacea, Agnus castus, Allium cepa, Hedera helix, Hippocastanus, Curcuma, Galphimia glauca ou Herba Thymi.

5. Procédé selon les revendications 1 à 4, dans lequel on prépare un extrait du mélange de 2 ou plusieurs essences.

6. Préparations pharmaceutiques avec une forte teneur en huiles essentielles et phénols, contenant un extrait d'essence, préparé d'après un procédé selon l'une des revendications 1 à 5.
